# EUROPEAN PATENT SPECIFICATION

(11) **EP 3 429 688 B1**
(45) Date of publication and mention of the grant of the patent: **06.05.2020**
(21) Application number: 17712066.4
(22) Date of filing: 15.03.2017
(51) Int. Cl.: A61N 5/10, A61B 8/12, A61B 34/20

(54) **BRACHYTHERAPY SYSTEM AND METHOD**
BRACHYTHERAPIESYSTEM UND VERFAHREN
SYSTÈME DE CURIETHÉRAPIE ET PROCÉDÉ

(30) Priority: 16.03.2016 IN 201641009101; 28.04.2016 EP 16167497
(43) Date of publication of application: 23.01.2019
(73) Proprietor: Koninklijke Philips N.V., 5656 AG Eindhoven (NL)
(72) Inventor: VAJINEPALLI, Pallavi, 5656 AE Eindhoven (NL); MALLYA, Yogisha, 5656 AE Eindhoven (NL); CHAKRABARTI, Biswaroop, 5656 AE Eindhoven (NL); SETH, Subhendu, 5656 AE Eindhoven (NL); SHARMA, Swetha, Badrinarayan, 5656 AE Eindhoven (NL); SHETTIGAR, Srikanth, 5656 AE Eindhoven (NL)
(74) Representative: van Iersel, Hannie
(86) International application number: PCT/EP2017/056120
(87) International publication number: WO 2017/158021

(56) References cited:
- WO-A1-2015/039995
- WO-A1-2015/181632

## Description

### FIELD OF THE INVENTION

The invention generally relates to the field of brachytherapy. More specifically, the invention is related to a brachytherapy system for supporting application of radiation to a body region of a patient body, to a method for operating the system and to a computer program for carrying out the method.

### BACKGROUND OF THE INVENTION

In brachytherapy, one or more radiation source(s) is/are placed inside a patient body within a treatment region comprising a tumor. Each radiation source emits ionizing radiation to treat the surrounding tissue with the main goal to destroy tumor cells included in this tissue. In one type of brachytherapy, which is usually called temporary brachytherapy, the radiation source(s) is/are placed within the treatment for defined shorter time intervals in order to apply a defined radiation dose particularly to the tumor cells. This type of brachytherapy allows for an efficient treatment of cervical cancer and also of other types of cancer, such as prostate cancer.

In order to place the radiation source within the treatment region, a so-called applicator is used. In order to prepare the radiation treatment, the applicator is inserted into the treatment region without including the radiation source(s). Once the applicator is correctly positioned, a dose calculation unit determines the dose distribution to be applied particularly on the basis of the relative position between the applicator and the tumor and on the basis of the anatomical configuration of the treatment region. Based on this dose distribution, an irradiation plan is then determined that specifies the positioning of the one or more radiation source(s) within the applicator and the treatment time such that the tumor cells are sufficiently treated and that sensitive tissue surrounding the tumor cells (also referred to as organs at risk) receives the lowest possible radiation dose. Thereupon, the radiation source(s) is/are delivered into the applicator in accordance with the irradiation plan.

In order to help guide the placement of the applicator, images of the treatment region are used which are acquired using an appropriate imaging modality. Moreover, the dose distribution and the irradiation plan are determined on the basis of an image acquired when the applicator is correctly positioned. In this regard, the preferred imaging modalities are computed tomography (CT) and magnetic resonance (MR) imaging, since these modalities allow for precisely determining the anatomical configuration of the treatment region and the position of the applicator. However, CT or MR imaging is often not available in hospitals due to the extensive costs of the required imaging equipment. In particular hospitals in developing countries do often not have CT or MR imaging equipment.

When CT or MR imaging is not available, the irradiation plan is often determined on the basis of several two-dimensional x-ray images, which may be acquired from different (orthogonal) directions. However, soft tissues, such as cervix, uterus, bladder, rectum etc., can usually not be seen in such images so that the dose calculation is made without taking into account the actual size and shape of the patient's anatomy. As a consequence, the irradiation plan may produce an under-dosage with respect to the tumor volume and/or may produce over-dosage with respect to organs at risk.

WO 2015/181632 discloses a system for generating a treatment plan for a brachytherapy treatment. In the system, an ultrasound image of an anatomical region including a treatment applicator is acquired. In addition, the location of the applicator is determined on the basis of tracking information provided by a tracking device comprising a marker which is attached to the applicator. On the basis of the tracking information, a graphical representation of the applicator is projected onto the ultrasound image. Further, a CT image of the anatomical region is obtained and the graphical representation of the applicator is projected onto the CT image. Thereupon, the ultrasound and CT images are combined by registering the graphical representations of the treatment applicator in the ultrasound and CT images in order to provide an improved visualization of the treatment tissues.

### SUMMARY OF THE INVENTION

Therefore, it is an object of the present invention to allow for a high quality brachytherapy treatment planning which can be implemented at lower costs compared with the treatment planning using MR or CT imaging.

In one aspect, the invention provides a brachytherapy system for supporting application of radiation to a body region of a patient body. The system cooperates with an applicator configured for fixating at least one radiation source in the body region. The system comprises an ultrasound (US) device configured for acquiring a three-dimensional image of the body region including the applicator, and an image generation unit. The image generation unit comprises a pre-stored three-dimensional model of the applicator and is configured to obtain position information relating the applicator on the basis of the three-dimensional image of the body region and to align the three-dimensional image of the body region and the pre-stored three-dimensional model of the applicator on the basis of the position information.

Since the system includes an US device for acquiring three-dimensional images of the body region instead of a CT or MR device, the system can be implemented at lower costs given the significantly lower costs of US devices. Moreover, by aligning the US image of the body region and the model of the applicator, the position of the applicator within the body region can be accurately determined, even if the applicator is not fully visible in the US image. As a consequence, it is in particular also possible to use applicators that are not specifically configured for US imaging (as it is typically the case for conventional applicators, which are usually configured for CT or MR imaging). The model of the applicator preferably comprises a suitable representation of the applicator in the image space so that the applicator can be aligned within the US image of the body region.

In one embodiment of the invention, the position information is determined on the basis of the three-dimensional image of the body region. In particular, a user of the system may mark points of the applicator within the three-dimensional image of the body region, and the system may determine the position information on the basis of these marked points. In order to allow for marking the points in the image of the body region, the system preferably comprises a display device for displaying the three-dimensional image of the body region to a user and an input device configured to receive user inputs for marking the predetermined points of the applicator within the displayed image.

In one embodiment, the position information corresponds to positions of predetermined points of the applicator marked within the image of the body region. These predetermined points may correspond to certain prominent points of the applicator, which can be easily identified in the image, such as, for example, the tip and points of connecting elements between components of the applicator.

A related embodiment provides that the model of the applicator comprises corresponding points and that the image generation unit is configured to align the image of the body region and the model of the applicator such that said points of the model of the applicator are arranged at the positions marked within the image of the body region.

In a further related embodiment, the three-dimensional model of the applicator comprises a set of plural predefined points, and wherein the position information correspond to positions of predefined points included in a subset of the set. Thus, the user of the system can select a subset of predefined points to be marked, e.g. in case some predefined points (not included in the selected subset) are not visible in the three-dimensional image of the body region.

In one embodiment, the position information correspond to one position of a predetermined point of the applicator marked within the image of the body region and a further point of the applicator marked with in the image of the body region, the predetermined point of the applicator corresponding to a predetermination point of the model of the applicator. It is an advantage of this embodiment that the user of the system does only have to mark one predetermined point of the applicator and can arbitrarily select a further point used for determining the position information.

In a related embodiment, the image generation unit is configured to determine an orientation of the applicator within the image from the marked points, and the image generation unit is configured to align the image of the body region and the model of the applicator such that said marked predetermined point of the applicator and the corresponding point of the model are arranged at the same position and that an orientation vector of the model is aligned with the determined orientation.

In one embodiment of the invention, the applicator comprises a plurality of components and the image generation unit is configured to obtain position in formation relating to each component and to align each corresponding component of the model of the applicator and the three-dimensional image of the body region on the basis of the position information associated with the component. Thus, different components of the applicator can be independently aligned with the image of the body region in this embodiment. Hereby, a more accurate alignment of the applicator model and the image of the body region can be achieved.

In order to enable the image generation unit to align one component of the applicator model with the image of the body region, the user may mark at least two predefined points of the component or one predefined point and a further point within the image of the body region, and the image generation may align the component of the applicator model using the marked points in a way described above.

In one embodiment of the invention, the brachytherapy system further comprises a display unit for displaying the model of the applicator and the image of the body region to a user and the image generation unit is further configured to move and/or rotate the model of the applicator relative to the image of the body region in response to input commands provided by a user. Hereby, the user can further adjust the model of the applicator within the image, e.g. in case the alignment on the basis of the position information does not lead to an optimal match between the model and the applicator depicted in the three-dimensional image of the body region.

In a further embodiment of the invention, the system further comprises a visualization unit configured to generate the three-dimensional image of the body region by stitching plural three-dimensional images acquired using the ultrasound device. This allows for generating an image of a body region which is larger than the field-of-view of the ultrasound device, which is usually not sufficiently large to acquire an image of the volume of interest.

In a further embodiment of the invention, the brachytherapy system further comprises a dose engine unit configured to calculate a radiation dose to be applied to the body region on the basis of the relative position of the model of the applicator and the image of the body region.

In a further aspect, the invention provides a method for operating a brachytherapy system for supporting the application of radiation to a body region of a patient body, the brachytherapy system cooperating with an applicator configured for fixating at least one radiation source in the body region and comprising an ultrasound device configured for acquiring a three-dimensional image of the body region including the applicator and an image generation unit including a pre-stored three-dimensional model of the applicator. The method comprises: (i) the image generation unit obtaining position information relating the applicator on the basis of the three-dimensional image of the body region, and (ii) the image generation unit aligning the three-dimensional image of the body region and the pre-stored three-dimensional model of the applicator on the basis of the position information.

In a further aspect of the invention, a computer program is provided. The computer program comprises program code means for instructing at least one processor to carry out the method, when the computer program is executed on the processor.

It shall be understood that the system of claim 1, the method of claim 12 and the computer program of claim 13 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 schematically and exemplarily shows a brachytherapy system according to an embodiment of the invention,
Fig. 2 schematically and exemplarily shows an applicator that can be used in the system,
Fig. 3 schematically and exemplarily shows a model of the applicator, and
Fig. 4 schematically and exemplarily illustrates the alignment of the model of the applicator and the depicted applicator in an image on the basis of an orientation vector.

### DETAILED DESCRIPTION OF EMBODIMENTS

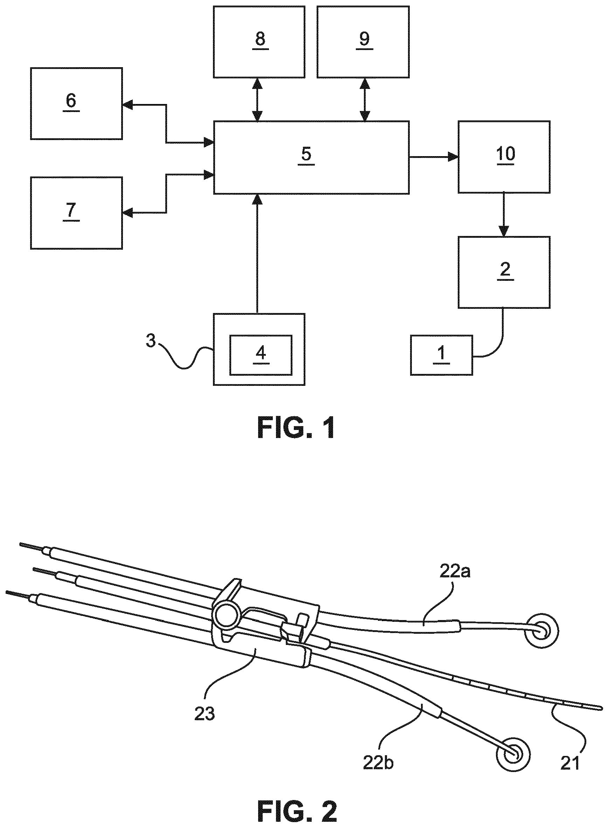

Fig. 1 schematically and exemplarily shows a temporal brachytherapy system for supporting application of radiation to a target region of a patient body, particularly in order to treat cancer. In one embodiment, the system is used for treating cervical cancer. In other embodiments, the system may be adapted to treat other types of cancer like endometrial cancer, for example.

The system cooperates with an applicator 1 for delivering one or more radiation source(s) to the target region. The applicator 1 includes one or more catheter(s) for receiving the radiation source(s) which may be configured as radioactive particles. Via the catheters, the radiation sources can be delivered to the target region and hold at defined positions (so-called dwell positions) within the catheters, where each catheter may provide several possible dwell positions. Before commencing the actual radiation treatment, the applicator 1 is appropriately positioned in the target region when it does not include the radiation source(s). On the basis of the position of the applicator relative to organs at risk (OARs) and to the target structure (e.g. a tumor) an irradiation plan is then determined in a way described in more detail herein below. The irradiation plan particularly defines the dwell positions of the radiation source(s) within the applicator and the irradiation time (often also referred to as dwell time). Then, the radiation source(s) are delivered into the applicator 1 and hold in place within the applicator 1 in accordance with the irradiation plan. In the embodiment illustrated in Fig. 1, the radiation source(s) are remotely delivered into the applicator 1 from an afterloader device 2. In further embodiments, the radiation source(s) can likewise be delivered manually into the applicator 1.

Fig. 2 schematically and exemplarily depicts an applicator 1 which can be used for a brachytherapy treatment of cervical cancer. The applicator 1 comprises a central tandem 21 and two ovoids 22a, 22b, which are connected by means of a joint element 23. The joint element 23 holds the tandem 21 and the ovoids 22a, 22b in an adjustable relative position. In use of the applicator 1 according to this embodiment, the tandem 21 can be inserted into the patient's uterus and the ovoids 22a, 22b may be positioned to flank the cervix. In further embodiments, the applicator 1 only comprises a tandem 21 or ovoids 22a, 22b. Likewise, the applicator 1 may not comprise a tandem 21 and/or ovoids 22a, 22, but may be configured in a different way. For instance, ring-tandem, interstitial or vaginal applicators may be used when treating cervical cancer. In general, the configuration of the applicator 1 may be configured in accordance with the target region of the patient body and, thus, may have other components in case it is used for treating other types of cancer than cervical cancer.

Further, the system comprises an US device 3 for acquiring three-dimensional images of the target region within the patient body (see Fig. 1). The US device 3 includes an US probe 4 which is configured for acquiring three-dimensional US images of a certain field-of-view and which may be a wobbler probe, for example. The US device 3 generates US image data, which are transmitted to a visualization unit 5. From the US image data, the visualization unit 5 can generate three-dimensional images of volumes of interest, for which the user of the system (typically a physician) has acquired US image data using the US probe 4. Moreover, the visualization unit 5 is preferably configured for automatically generating three-dimensional US images of the complete target region, by combining overlapping US images of several smaller volumes (so-called stitching). It is thus possible to generate a three-dimensional image of a volume of interest that is larger than the field-of-view of the US probe 4, which field-of-view is usually relatively small.

Preferably, the visualization unit 5 carries out an automatic stitching procedure, which combines the US images without requiring a user input. In principle, any automatic stitching algorithm known to the person skilled in art can be applied in the visualization unit 5 for this purpose. One exemplary algorithm, which is applicable in the visualization unit, is described in R. Dalvi et al., "3D Ultrasound Volume Stitching Using Phase Symmetry and Harris Corner Detection for Orthopaedic Applications", SPIE medical imaging (p. 762330). In a further exemplary algorithm, the visualization unit 5 firstly segments the US images so that these images contain borders of anatomical structures. Then, the segmented images are combined using an intensity-based registration method. In this method, the normalized cross-correlation may be used as a metric to obtain a transform that maps a first image to a second image such that common features overlap. This transform is applied to the first image in order to combine the images such that they are in alignment to each other.

Three-dimensional US images generated in the visualization unit 5 can be displayed to the user of the system by means of a display device 6. Further, the system includes input means 7 allowing the user to interact with the system and particularly with the views presented to the user at the display device 6. The input means 7 may particularly comprise a keyboard and a pointing device such as a computer mouse or a trackpad.

Moreover, the system includes an organ segmentation unit 8, which is configured to identify and delineate the target structure of the radiation treatment, i.e. a tumor, and further structures of interest in the three-dimensional US images. The further structures of interest may particularly include the OARs, which shall receive a low radiation dose during the radiation treatment. In case of a brachytherapy treatment of cervical cancer, such OARs may particularly comprise the uterus, the cervix, the bladder and the rectum. In order to identify and delineate the target structure and the further structures, an automatic or semi-automatic image-processing-based algorithm may be used. In an automatic procedure, the organ segmentation unit 8 may automatically delineate the relevant structures using suitable image recognition techniques. In a semi-automatic procedure, the user may mark points and/or parts of the contour of the relevant structures, and the organ segmentation unit 8 may delineate the complete structures on the basis of the user-defined portions using suitable image recognition techniques.

As will be explained herein below in more detail, the system further comprises an applicator reconstruction unit 9, which is configured to position a three-dimensional model 31 of the applicator 1 relative to the three-dimensional US images of the target region in a semi-automatic procedure. Hereby, the applicator reconstruction unit 9 aligns the model 31 of the applicator 1 and the US image of the target region such that the relative position of the model 31 of the applicator 1 with respect to the image volume of the target region approximately corresponds to the relative position of the applicator 1 and the target region.

On this basis and on the basis of the delineations of the target structures and the further relevant structures, such as the OARs, in the US image of the target region, a dose engine unit 10 generates the irradiation plan for the radiation treatment of the target structure. As explained above, the irradiation plan specifies the number of radiation source(s) and their strengths, the dwell positions of the radiation sources (i.e. their loading pattern) within the applicator 1, and the dwell time(s) for the radiation source(s). The dose engine unit 10 generates the irradiation plan such that a sufficient radiation dose is delivered to the target structure, which allows for efficiently treating the target structure. At the same time, the irradiation plan is generated in such a way that the OARs (e.g. the rectum and bladder in case of a treatment of cervical cancer) receive the lowest possible radiation dose. For generating the irradiation plan in such a way, the dose engine unit 10 may use any suitable treatment planning algorithm known to a person skilled in the art. In one embodiment, the treatment planning algorithm may particularly be based on the TG43U1S1 protocol as recommended by the American Association of Physicists in Medicine (AAPM), see AAPM Report No. 51, "Dosimetry of Interstitial Brachytherapy Sources", International Standard Book Number: 1-56396-462-7.

The visualization unit 5, the organ segmentation unit 8, the applicator reconstruction unit 9 and the dose engine unit 10 may be implemented as software applications. These software applications may be parts of an integrated computer program, or they may be implemented as separate computer programs. Particularly in case the software applications are configured as a single integrated computer program, they may be executed in a single computer device. Likewise, the software applications can be executed on two or more computers, which are suitably connected to each other, e.g. via a direct connection or a network connection. The display device 6 and the input means 7 may be integrated into (one of) the computer device(s) executing the software applications, and the US device 3 may likewise be coupled to (one of) the computer device(s). Likewise, the display device 6 and the input means 7 may be part of a separate device, such as a further computer device, which does not execute one of the software applications and which is connected to (one of) the computer device(s) executing the software applications. Hereby, it is particularly possible to control the system and inspect its output views from a remote location.

The model 31 of the applicator 1 is pre-stored in the applicator reconstruction unit 9 and loaded when a radiation treatment is to be carried out using the applicator 1. In this regard, it is preferably possible to store in the applicator reconstruction unit 9 models 31 of plural applicators 1 and to select the model 31 of the used applicator 1 each time a radiation treatment is to be planned. Thus, it is possible to generate irradiation plans for different applicators 1 used in the brachytherapy treatment carried out using the brachytherapy system. Moreover, the applicator reconstruction unit 9 preferably allows users of the brachytherapy system to install models 31 of applicators 1 at the operating site of the brachytherapy system. Thus, it is possible to install new models 31 of applicators 1 when new applicators 1 are to be used in the brachytherapy system.

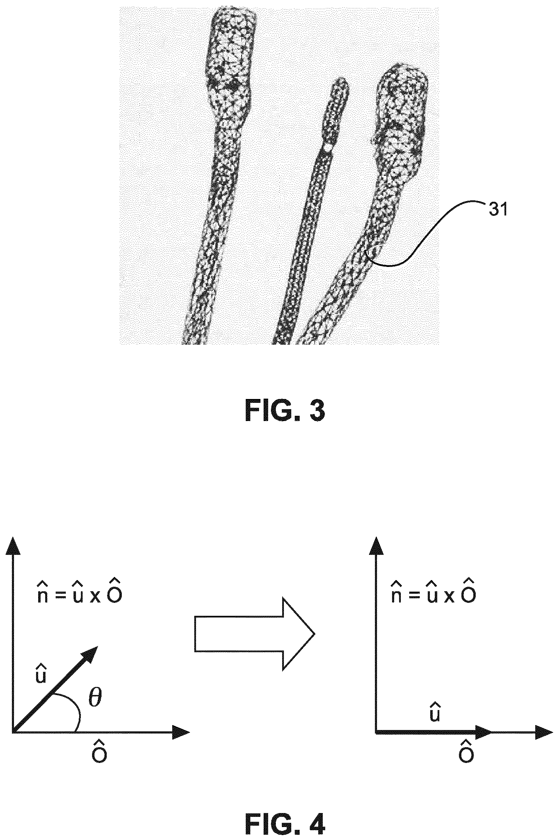

The models 31 of the applicators 1 preferably comprise a three-dimensional representation of the contour of the applicator 1 in the three-dimensional image space. In this regard, Fig. 3 schematically and exemplarily illustrates a model 31 of the applicator 1 described above and shown in Fig. 2. More specifically, Fig. 3 shows a model comprising the components of the applicator 1 (the tandem 21 and the ovoids 22a and 22b), which may be aligned with the image of the body region separately as will be explained herein below. In order to create a model 31 of an applicator 1, a three-dimensional of the applicator 1 may be acquired with a suitably high resolution (e.g. 0.5 mm in x- and y-direction and 1.5 mm in z-direction). The three-dimensional image of the applicator 1 can be acquired using any suitable imaging modality. In one embodiment, the three-dimensional image of the applicator 1 is a CT image acquired using a CT device. In further embodiments, the three-dimensional image is a magnetic resonance image acquired using an MR device. In these embodiments, an MR-compatible applicator 1 is used, i.e. an applicator 1 which is made of a material that can be seen in MR images. In the three-dimensional image, the contour of the applicator 1 may be delineated using a suitable automatic, semi-automatic or manual delineation procedure. From the delineated contour, the model 31 of the applicator can be generated and stored in any suitable format, which allows overlaying the model 31 and the three-dimensional image of the target region. For instance, the model 31 may be stored as an image, a binary mask, a surface mesh model or a contour model (i.e. as a set of contours). In case a surface mesh is used, a triangulated surface mesh may be generated from the delineated contour of the applicator 1 using a suitable triangulation algorithm.

As mentioned above, the model 31 of the applicator is aligned with the US image of the target region as a whole or individual components of the applicator model 31 are separately aligned with the US image of the target region. When using the applicator 1 described above, such components may correspond to the tandem 21 and the ovoids 22a, 22b, for example. The alignment of the applicator model 31 or its components may be made on the basis of one or more predefined anchor points of the model 31 or the components. These anchor points correspond to predetermined landmark points of the real applicator 1, which are preferably selected such that the user of the brachytherapy system can easily identify these landmark points in the US images. For the applicator 1 depicted in Fig. 2, these landmark points may correspond to the tip of the central tandem 21, the end points of the ovoids and the midpoint of the joint element 23, for example. Moreover, further landmark points may be defined, such as points where the diameter of the central tandem 21 or an ovoid 22a, 22b changes.

Using such a model 31 of an applicator 1, the treatment planning procedure is carried out in the following manner in one embodiment of the invention:
Firstly, the user of the brachytherapy places the applicator 1 within the target region of the patient's body. Simultaneously, the user may use the US probe 4 for acquiring US images of the target region showing the inserted applicator 1. These images are preferably displayed at the display device 6 substantially in real time in order to assist the user in placing the applicator 1 at the appropriate position. Once the applicator 1 is placed at the appropriate position for carrying out the brachytherapy treatment, the user uses the US probe 4 for acquiring a series of three-dimensional US images of the volume of interest showing the target structure to be treated (e.g. a tumor), the applicator 1, and further relevant anatomical structures, such as OARs in the vicinity of the tumor and the applicator 1. Using these US images, the visualization unit 5 generates a single image of the target region by stitching the acquired US images, and this image is presented to the user at the display device 6. Thus, the user can view the complete target region including all relevant anatomical structures and the positioned applicator 1 in a single image volume.

In a first embodiment, the user may then mark those landmark points of the used applicator 1 within the image volume, which are visible in the image. Since the applicator 1 is a rigid structure, the user may mark at least two visible landmark points. However, it may be preferred that the user marks all visible landmark points in order to improve the accuracy of the subsequent registration procedure. These may be all landmark points predefined for the applicator 1. However, particularly when the applicator 1 is not specifically configured for US imaging, only a subset of the predefined landmark points may be visible in the US image, and the user may mark the landmark points of this subset.

For marking a landmark point, the user may e.g. move a pointer or cursor within the displayed image using the input means 7 and may input a control command, such as e.g. a "click", when the pointer or cursor is positioned at the landmark point. Moreover, the user may identify each marked landmark point by means of the input means 7. For this purpose, a list of all predetermined landmark points of the used applicator may be presented to the user at the display device 6 (after the user has specified the used applicator 1), and each time the user marks a landmark point, the user may select the list entry corresponding to the marked landmark point from the list.

The positions of the marked landmark points are provided to the applicator reconstruction unit 9. The applicator reconstruction unit 9 identifies the anchor points of the model 31 of the applicator 1 that correspond to the marked landmark points. Then, the applicator reconstruction unit 9 combines the model 31 of the applicator 1 and the US image volume of the target region and performs an image registration procedure in such a way that each identified anchor point of the model 31 of the applicator 1 is arranged substantially at the position of the corresponding landmark point of the applicator marked in the image. For this purpose, the applicator reconstruction 9 determines a suitable transformation of the model 31, which transforms the model 31 to achieve the aforementioned result.

In one embodiment, one rigid transformation for the complete model 31 of the applicator 1 is determined. In further embodiments, predetermined portions of the model 31 of the applicator 1 are transformed separately. In particular, those portions of the model 31 may be transformed separately which correspond to components of the applicator 1 that can be moved relative to each other. Hereby, it is possible to perform an accurate registration of the model 31 for different relative positions of the movable components of the applicator 1. With respect to model 31 of the applicator 1 shown in Fig. 2, the applicator reconstruction unit 1 may separately transform the central tandem 21 and the ovoids 22a, 22b, for example, since these components of the applicator 1 can be moved relative to each other. In case different portions of the model 31 of the applicator 1 are transformed separately, the user has to mark at least two landmark points for each component of the applicator 31 that corresponds to a separately transformed portion of the model 31 of the applicator.

In a further embodiment, the user does only have to mark one landmark point of the applicator 1 or a component thereof in the acquired three-dimensional US image. In the following an individual alignment of the components of the applicator is assumed. In this case, the user marks one landmark point and a further arbitrarily selected point of each component of the applicator 1. In this regard, each component of the applicator model 31 may include only one landmark point. However, it is likewise possible that one or more components of the applicator model 31 include(s) several landmark points. In this case, the user can select the landmark point to be marked among several possible landmark points.

On the basis of the marked landmark point and the additionally marked point of one component of the applicator, the visualization unit 5 determines the orientation of the component in the US image. In particular, this orientation can be parameterized by means of a three-dimensional orientation vector connecting the marked landmark point and the marked additional point. On the basis of this orientation and on the basis of the marked landmark point, the applicator reconstruction unit 9 aligns the component of the applicator model 31 with the corresponding component of the real applicator 1 shown in the US image.

For this purpose, the applicator reconstruction unit 9 positions the component of the model 31 in the US image such that the marked landmark point and the corresponding landmark point of the model 13 are located at the same positions. Further, the component of the applicator model 31 is rotated such that the orientation of the model 31 corresponds to the orientation of the applicator component within the US image. In order to perform this rotation, the visualization unit 5 computes a rotation axis and an angle between the aforementioned orientation vector derived from the marked points in the image and a corresponding vector of the unaligned model 31 of the applicator component with respect to the rotation axis. As schematically and exemplarily illustrated in Fig. 4 (left-hand side), the rotation axis *n̂* may be computed as the cross product of the orientation vector *Ô* (derived from the marked points in the image as described above) and the corresponding vector *û* of the unaligned component of the model 31, i.e.*n̂* = *û* × *Ô*. The determined angle *θ* corresponds to the angle between the vectors *Ô* and *û* in a plane perpendicular to the rotation axis *n̂*. In order to align the component of the applicator model 31 and the depiction of the applicator component in the acquired US image, the component of the applicator model 31 is then rotated by the angle *θ* around the rotation axis *n̂* (as shown on the righthand side of Fig. 4).

In such a way, an accurate alignment of the component of the applicator model 31 and the applicator component shown in the US image of the target region can be achieved, when the points marked by the user reside on the medial line of the applicator component. Thus, the user preferably selects the further point which is to be marked in addition to the predefined landmark point in such a way that both points reside on the medial line. In a variation of the aforementioned embodiment, the applicator reconstruction unit 9 may not individually align each component of the applicator model 31 with the US image, but may align the applicator model 31 as a whole with the US image. For this purpose, the user may mark one landmark point and one further point of one component of the applicator and this component may be aligned with the US image in the way described above. The further components may be aligned in connection with this component on the basis a rigid transformation of the applicator model 31.

Upon having aligned the applicator model 31 with the US image, the applicator reconstruction unit 9 preferably generates a combined image in which the aligned applicator model 31 is overlaid over the US image, and this combined image may be presented to the user of the brachytherapy system by means of the display device 6. Optionally, the applicator reconstruction unit 9 may then be operated in an editing mode in which the user can move and/or the model 31 included in the US image volume, where moving the model 31 may comprise displacing the model 31 relative to the US image volume of the target region in any direction and rotating the model 31 may comprise rotations around any axis. When the user modifies the relative position of the applicator model 31 and the US image in such a way, the modified relative position is used in the next steps of the planning procedure instead of the relative position between the applicator model 31 and the US image resulting from the registration procedure carried in the applicator reconstruction unit 9.

In a further step of the treatment planning procedure, the organ segmentation unit 8 identifies and delineates the target structure to be treated and further relevant anatomical structures within the target region, such as organs at risk, shown in the US image in a way described above. This step may be performed before the US image is aligned with the model 31 of the applicator or thereafter. In any case, a three-dimensional image of the target region is generated in the aforementioned steps, which includes the contours of the target structures and further relevant anatomical structures and which includes the model 31 of the applicator 1 which is correctly positioned relative to the target structure and the other structures within the target region.

On the basis of this image, the dose engine unit 10 determines the irradiation plan for the brachytherapy treatment and, then, the brachytherapy treatment is carried out in accordance with the treatment plan.

Variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

## Claims

1. A brachytherapy system for supporting application of radiation to a body region of a patient body, the system being configured to cooperate with an applicator (1) configured for fixating at least one radiation source in the body region and the system comprising:
an ultrasound device (3) configured for acquiring a three-dimensional image of the body region including the applicator (1), and
an image generation unit (9) comprising a pre-stored three-dimensional model (31) of the applicator (1) and being configured to obtain position information relating the applicator (1) on the basis of the three-dimensional image of the body region and to align the three-dimensional image of the body region and the pre-stored three-dimensional model (31) of the applicator (1) on the basis of the position information.

2. The brachytherapy system as defined in claim 1, wherein the position information corresponds to positions of predetermined points of the applicator (1) marked within the image of the body region.

3. The brachytherapy system as defined in claim 2, wherein the model (31) of the applicator (1) comprises corresponding points and wherein the image generation unit (9) is configured to align the image of the body region and the model (31) of the applicator (1) such that said points of the model (31) of the applicator (1) are arranged at the positions marked within the image of the body region.

4. The brachytherapy system as defined in claim 2, wherein the three-dimensional model (31) of the applicator (1) comprises a set of plural predefined points, and wherein the position information correspond to positions of predefined points included in a subset of the set.

5. The brachytherapy system as defined in claim 1, wherein the position information correspond to one position of a predetermined point of the applicator (1) marked within the image of the body region and a further point of the applicator (1) marked with in the image of the body region, the predetermined point of the applicator (1) corresponding to a predetermination point of the model (31) of the applicator (1).

6. The brachytherapy system as defined in claim 1, wherein the image generation unit (9) is configured to determine an orientation of the applicator (1) within the image from the marked points, and wherein the image generation unit (9) is configured to align the image of the body region and the model (31) of the applicator (1) such that said marked predetermined point of the applicator (1) and the corresponding point of the model (31) are arranged at the same position and that an orientation vector of the model (31) is aligned with the determined orientation.

7. The brachytherapy system as defined in claim 2, further comprising a display device (6) for displaying the three-dimensional image of the body region to a user and an input device (7) configured to receive user inputs for marking the predetermined points of the applicator (1) within the displayed image.

8. The brachytherapy system as defined in claim 1, wherein the applicator (1) comprises a plurality of components (21; 22a; 22b) and the image generation unit (9) is configured to obtain position information relating to each component (21; 22a; 22b) and to align each corresponding component of the model (31) of the applicator (1) and the three-dimensional image of the body region on the basis of the position information associated with the component.

9. The brachytherapy system as defined in claim 1, further comprising a display unit (6) for displaying the model (31) of the applicator (1) and the image of the body region to a user, wherein the image generation unit (9) is further configured to move and/or rotate the model (31) of the applicator (1) relative to the image of the body region in response to input commands provided by a user.

10. The brachytherapy system as defined in claim 1, further comprising a visualization unit (5) configured to generate the three-dimensional image of the body region by stitching plural three-dimensional images acquired using the ultrasound device (3).

11. The brachytherapy system as defined in claim 1, further comprising a dose engine unit (10) configured to calculate a radiation dose to be applied to the body region on the basis of the relative position of the model (31) of the applicator (1) and the image of the body region.

12. A method for operating a brachytherapy system for supporting the application of radiation to a body region of a patient body, the brachytherapy system cooperating with an applicator (1) configured for fixating at least one radiation source in the body region and comprising an ultrasound device (3) configured for acquiring a three-dimensional image of the body region including the applicator (1) and an image generation unit (9) including a pre-stored three-dimensional model (31) of the applicator (1), the method comprising:
the image generation unit (9) obtaining position information relating the applicator (1) on the basis of the three-dimensional image of the body region, and
the image generation unit (9) aligning the three-dimensional image of the body region and the pre-stored three-dimensional model (31) of the applicator (1) on the basis of the position information.

13. A computer program comprising program code means for instructing at least one processor to carry out a method as defined in claim 12, when the computer program is executed on the processor.

## Patentansprüche

1. Ein Brachytherapiesystem, mit dem eine Körperregion des Patienten einer Strahlung ausgesetzt wird, wobei das System so konfiguriert ist, dass es mit einem Applikationsgerät (1) zusammenwirkt, das mindestens eine Strahlungsquelle in der Körperregion anbringt. Hierbei besteht das System aus Folgendem:
einem Ultraschallgerät (3), das ein dreidimensionales Bild der Körperregion einschließlich des Applikationsgeräts (1) erfasst, und
einem Bildgebungsgerät (9), das über ein vorab gespeichertes dreidimensionales Modell (31) des Applikationsgeräts (1) verfügt und so konfiguriert ist, dass es die Positionsdaten des Applikationsgeräts (1) auf Grundlage des dreidimensionalen Bilds der Körperregion erfasst und das dreidimensionale Bild der Körperregion und das vorab gespeicherte dreidimensionale Modell (31) des Applikationsgeräts (1) auf Grundlage der Positionsdaten ausrichtet.

2. Das Brachytherapiesystem gemäß Anspruch 1, wobei die Positionsdaten den Positionen der vordefinierten Punkte des Applikationsgeräts (1) entsprechen, die im Bild der Körperregion gekennzeichnet sind.

3. Das Brachytherapiesystem gemäß Anspruch 2, wobei das Modell (31) des Applikationsgeräts (1) übereinstimmende Punkte umfasst und das Bildgebungsgerät (9) so konfiguriert ist, dass es das Bild der Körperregion und das Modell (31) des Applikationsgeräts (1) so ausrichtet, dass sich die besagten Punkte des Modells (31) des Applikationsgeräts (1) an den im Bild der Körperregion gekennzeichneten Positionen befinden.

4. Das Brachytherapiesystem gemäß Anspruch 2, wobei das dreidimensionale Modell (31) des Applikationsgeräts (1) einen Satz mit mehreren vordefinierten Punkte umfasst und die Positionsdaten den Positionen der vordefinierten Punkte eines Untersatzes des Satzes entsprechen.

5. Das Brachytherapiesystem gemäß Anspruch 1, wobei die Positionsdaten der Position eines vordefinierten, im Bild der Körperregion gekennzeichneten Punkts des Applikationsgeräts (1) sowie eines weiteren im Bild der Körperregion gekennzeichneten Punkts des Applikationsgeräts (1) entsprechen, wobei der vordefinierte Punkt des Applikationsgeräts (1) einem vordefinierten Punkt des Modells (31) des Applikationsgeräts (1) entspricht.

6. Das Brachytherapiesystem gemäß Anspruch 1, wobei das Bildgebungsgerät (9) so konfiguriert ist, dass es die Ausrichtung des Applikationsgeräts (1) innerhalb des Bilds anhand der gekennzeichneten Punkte ermittelt. Zudem ist das Bildgebungsgerät (9) so konfiguriert, dass es das Bild der Körperregion und das Modell (31) des Applikationsgeräts (1) so ausrichtet, dass sich die besagten vordefinierten Punkte des Applikationsgeräts (1) und des Modells (31) an derselben Position befinden, und dass sich der Ausrichtungsvektor des Modells (31) an der festgelegten Ausrichtung orientiert.

7. Das Brachytherapiesystem gemäß Anspruch 2, das zudem ein Anzeigegerät (6) zum Anzeigen des dreidimensionalen Bilds der Körperregion für den Benutzer sowie ein Eingabegerät (7) umfasst, mit dem Benutzereingaben zum Kennzeichnen der vordefinierten Punkte des Applikationsgeräts (1) auf dem angezeigten Bild möglich sind.

8. Das Brachytherapiesystem gemäß Anspruch 1, wobei das Applikationsgerät (1) mehrere Komponenten (21; 22a; 22b) umfasst und das Bildgebungsgerät (9) so konfiguriert ist, dass es die Positionsdaten der einzelnen Komponenten (21; 22a; 22b) abrufen und die jeweils entsprechenden Komponenten des Modells (31) des Applikationsgeräts (1) und das dreidimensionale Bild der Körperregion auf Grundlage der den Komponenten zugeordneten Positionsdaten ausrichten kann.

9. Das Brachytherapiesystem gemäß Anspruch 1, das zudem ein Anzeigegerät (6) zum Anzeigen des Modells (31) des Applikationsgeräts (1) und des Bilds der Körperregion umfasst, wobei das Bildgebungsgerät (9) zudem so konfiguriert ist, dass es das Modell (31) des Applikationsgeräts (1) relativ zum Bild der Körperregion dreht und/oder bewegt, wenn es vom Benutzer entsprechende Eingabebefehle erhält.

10. Das Brachytherapiesystem gemäß Anspruch 1, das zudem ein Visualisierungsgerät (5) umfasst, dass so konfiguriert ist, dass es ein dreidimensionales Bild der Körperregion generiert, indem es mehrere, vom Ultraschallgerät (3) erhaltene dreidimensionale Bilder zusammenführt.

11. Das Brachytherapiesystem gemäß Anspruch 1, das zudem ein Dosierungsgerät (10) umfasst, das so konfiguriert ist, dass es die auf die Körperregion anzuwendende Strahlungsdosis auf Grundlage der relativen Position des Modells (31) des Applikationsgeräts (1) sowie des Bilds der Körperregion berechnet.

12. Ein Methode zum Bedienen eines Brachytherapiesystems, mit dem eine Körperregion des Patienten einer Strahlung ausgesetzt wird, wobei das Brachytherapiesystem mit einem Applikationsgerät (1) zusammenwirkt, das mindestens eine Strahlungsquelle in der Körperregion anbringt. Hierbei besteht das System aus einem Ultraschallgerät (3), das ein dreidimensionales Bild der Körperregion einschließlich des Applikationsgeräts (1) erfasst, und einem Bildgebungsgerät (9), das über ein vorab gespeichertes dreidimensionales Modell (31) des Applikationsgeräts (1) verfügt. Die Methode besteht hierbei aus folgenden Elementen:
einem Bildgebungsgerät (9), das auf Grundlage des dreidimensionalen Bilds der Körperregion Positionsdaten für das Applikationsgerät (1) erfasst, und
einem Bildgebungsgerät (9), das das dreidimensionale Bild der Körperregion und das vorab gespeicherte dreidimensionale Modell (31) des Applikationsgeräts (1) auf Grundlage der Positionsdaten ausrichtet.

13. Ein Computerprogramm, das Programmcode umfasst, mit dem mindestens ein Prozessor die in Anspruch 12 angeführte Methode durchführt, wenn das Computerprogramm auf dem Prozessor ausgeführt wird.

## Revendications

1. Système de brachythérapie destiné à l'aide à l'application d'un rayonnement dans une zone corporelle d'un corps de patient, ledit système étant conçu pour coopérer avec un applicateur (1) conçu pour fixer au moins une source de rayonnement dans la zone corporelle et ledit système comprenant:
un dispositif à ultrasons (3), conçu pour acquérir une image tridimensionnelle de la zone corporelle, comprenant l'applicateur (1) et
une unité de génération d'image (9) comprenant un modèle (31) tridimensionnel préenregistré de l'applicateur (1) et étant conçue pour obtenir des informations de position relatives à l'applicateur (1) en fonction de l'image tridimensionnelle de la zone corporelle et pour aligner l'image tridimensionnelle de la zone corporelle et le modèle (31) tridimensionnel préenregistré de l'applicateur (1) en fonction des informations de position.

2. Système de brachythérapie selon la revendication 1, dans lequel les informations de position correspondent à des positions des points prédéterminés de l'applicateur (1) marqués à l'intérieur de l'image de la zone corporelle.

3. Système de brachythérapie selon la revendication 2, dans lequel le modèle (31) de l'applicateur (1) comprend des points correspondants et dans lequel l'unité de génération d'image (9) est conçue pour aligner l'image de la zone corporelle et le modèle (31) de l'applicateur (1) de telle sorte que lesdits points du modèle (31) de l'applicateur (1) sont disposés aux positions marquées à l'intérieur de l'image de la zone corporelle.

4. Système de brachythérapie selon la revendication 2, dans lequel le modèle (31) tridimensionnel de l'applicateur (1) comprend un ensemble de plusieurs points prédéfinis, et
dans lequel les informations de position correspondent à des positions de points prédéfinis inclus dans un sous-ensemble de l'ensemble.

5. Système de brachythérapie selon la revendication 1, dans lequel les informations de position correspondent à une position d'un point prédéterminé de l'applicateur (1) marqué à l'intérieur de l'image de la zone corporelle et à un autre point de l'applicateur (1) marqué dans l'image de la zone corporelle, le point prédéterminé de l'applicateur (1) correspondant à un point de prédétermination du modèle (31) de l'applicateur (1).

6. Système de brachythérapie selon la revendication 1, dans lequel l'unité de génération d'image (9) est conçue pour déterminer une orientation de l'applicateur (1) à l'intérieur de l'image à partir des points marqués, et dans lequel l'unité de génération d'image (9) est conçue pour aligner l'image de la zone corporelle et du modèle (31) de l'applicateur (1) de telle sorte que ledit point prédéterminé marqué de l'applicateur (1) et le point correspondant du modèle (31) sont disposés à la même position et qu'un vecteur d'orientation du modèle (31) est aligné avec l'orientation déterminée.

7. Système de brachythérapie selon la revendication 2, comprenant en outre un dispositif d'affichage (6) pour afficher l'image tridimensionnelle de la zone corporelle à un utilisateur et un dispositif d'entrée (7) conçu pour recevoir des entrées utilisateur pour marquer les points prédéterminés de l'applicateur (1) dans l'image affichée.

8. Système de brachythérapie selon la revendication 1, dans lequel l'applicateur (1) comprend une pluralité de composants (21; 22a; 22b) et l'unité de génération d'image (9) est conçue pour obtenir des informations de position relatives à chaque composant (21; 22a; 22b) et pour aligner chaque composant correspondant du modèle (31) de l'applicateur (1) et l'image tridimensionnelle de la zone corporelle en fonction des informations de position associées au composant.

9. Système de brachythérapie selon la revendication 1, comprenant en outre une unité d'affichage (6) pour afficher le modèle (31) de l'applicateur (1) et l'image de la zone corporelle à un utilisateur, dans lequel l'unité de génération d'image (9) est conçue en outre pour déplacer et/ou pour tourner le modèle (31) de l'applicateur (1) par rapport à l'image de la zone corporelle en réponse à des commandes d'entrée fournies par un utilisateur.

10. Système de brachythérapie selon la revendication 1, comprenant en outre une unité de visualisation (5) conçue pour générer l'image tridimensionnelle de la zone corporelle par assemblage d'une pluralité d'images tridimensionnelles acquises à l'aide du dispositif à ultrasons (3).

11. Système de brachythérapie selon la revendication 1, comprenant en outre une unité de moteur de dosage (10) conçue pour calculer une dose de rayonnement à appliquer à la zone corporelle en fonction de la position relative du modèle (31) de l'applicateur (1) et de l'image de la zone corporelle.

12. Procédé de fonctionnement d'un système de brachythérapie destiné à l'aide à l'application d'un rayonnement à une zone corporelle d'un corps de patient, ledit système de brachythérapie coopérant avec un applicateur (1) conçu pour fixer au moins une source de rayonnement dans la zone corporelle et comprenant un dispositif à ultrasons (3), conçu pour acquérir une image tridimensionnelle de la zone corporelle, comprenant l'applicateur (1) et une unité de génération d'image (9) comprenant un modèle (31) tridimensionnel préenregistré de l'applicateur (1), ledit procédé comprenant:
l'unité de génération d'image (9) obtenant des informations de position concernant l'applicateur (1) en fonction de l'image tridimensionnelle de la zone corporelle; et
l'unité de génération d'image (9) alignant l'image tridimensionnelle de la zone corporelle et le modèle (31) tridimensionnel préenregistré de l'applicateur (1) en fonction des informations de position.

13. Programme informatique comprenant des moyens de code programme permettant de donner des instructions à au moins un processeur pour mettre en œuvre un procédé selon la revendication 12, lorsque le programme informatique est exécuté sur le processeur.
